(19) **Europäisches Patentamt European Patent Office Office européen des brevets**

(11) **EP 1 864 683 B1**

(12) **EUROPEAN PATENT SPECIFICATION**

(45) Date of publication and mention of the grant of the patent:
**01.09.2010 Bulletin 2010/35**

(51) Int Cl.:
*A61K 31/513* (2006.01)   *A61K 31/44* (2006.01)
*A61P 35/00* (2006.01)   *A61K 41/00* (2006.01)

(21) Application number: **06730907.0**

(22) Date of filing: **31.03.2006**

(86) International application number:
**PCT/JP2006/306959**

(87) International publication number:
**WO 2006/106983 (12.10.2006 Gazette 2006/41)**

(54) **RADIOTHERAPY ENHANCER**

STRAHLENTHERAPIEVERSTÄRKER

AGENT RADIOSENSIBILISANT

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.04.2005 JP 2005105819**

(43) Date of publication of application:
**12.12.2007 Bulletin 2007/50**

(73) Proprietor: **Taiho Pharmaceutical Co., Ltd. Chiyoda-ku, Tokyo 1018444 (JP)**

(72) Inventor: **FUKUSHIMA, Masakazu Taiho Pharmaceutical Co., Ltd. Tokushima--shi, Tokushima 7310132 (JP)**

(74) Representative: **Hartz, Nikolai et al Wächtershäuser & Hartz Patentanwaltspartnerschaft Weinstrasse 8 80333 München (DE)**

(56) References cited:

- **HARADA K ET AL: "Combined effects of the oral fluoropyrimidine anticancer agent, S-1 and radiation on human oral cancer cells" ORAL ONCOLOGY, ELSEVIER SCIENCE, OXFORD, GB, vol. 40, no. 7, 1 August 2004 (2004-08-01), pages 713-719, XP004511987 ISSN: 1368-8375**

- **IWASE H ET AL: "Esophageal cancer with colonic metastasis successfully treated by chemoradiotherapy followed by chemotherapy with S-1 and cisplatin" INTERNATIONAL JOURNAL OF CLINICAL ONCOLOGY, CHURCHILL LIVINGSTONE JAPAN, TOKYO, GB, vol. 9, 1 January 2004 (2004-01-01), pages 398-402, XP003000958 ISSN: 1341-9625**

- **TAKAHASHI T ET AL: "Histological Complete Response in a Case of Advanced Gastric Cancer Treated by Chemotherapy with S-1 Plus Low-dose Cisplatin and Radiation" JAPANESE JOURNAL OF CLINICAL ONCOLOGY, TOKYO, JP, vol. 33, no. 11, 1 January 2003 (2003-01-01), pages 584-588, XP003000956 ISSN: 0368-2811**

- **KATAKURA, YOSHIKI ET AL: "Assessment and strategy of treatment for inoperable pancreatic cancer" SUIZO , 20(4), 349-356 CODEN: SUIZEH; ISSN: 0913-0071, 2005, XP002502076**

- **MOERTEL C G ET AL: "Therapy of locally unresectable pancreatic carcinoma: A randomized comparison of high dose (6000 rads) radiation alone, moderate dose radiation (4000 rads + 5-fluorouracil), and high dose radiation + 5-fluorouracil. The gastrointestinal tumor study group" CANCER 1981 US, vol. 48, no. 8, 1981, pages 1705-1710, XP008098150 ISSN: 0008-543X**

- **KELLY S A ET AL: "The use of simultaneous radiotherapy and 5-flurouracil in patients with inoperable squamous cell lung cancer" CLINICAL RADIOLOGY 1989 GB, vol. 40, no. 3, 1989, pages 311-313, XP002502077 ISSN: 0009-9260**

**(Cont. next page)**

- HARADA K. ET AL.: 'Combined effects of the oral fluoropyrimidine anticancer agent, S-1 and radiation on human oral cancer cells' ORAL ONCOLOGY vol. 40, 2004, pages 713 - 719, XP004511987
- IWASE H. ET AL.: 'Esophageal cancer with colonic metastasis successfully treated by chemoradiotherapy followed by chemotherapy with S-1 and cisplatin' INT. J. CLIN. ONCOL. vol. 9, 2004, pages 398 - 402, XP003000958
- TAKAHASHI T. ET AL.: 'Histological Complete Response in a Case of Advanced Gastric Cancer Treated by Chemotherapy with S-1 Plus Low-dose Cisplatin and Radiation' JPN. J. CLIN. ONCOL. vol. 33, no. 11, 2003, pages 584 - 588, XP003000956
- ICHINOSE Y. ET AL.: 'S-1 Plus Cisplatin Combination Chemotherapy in Patients with Advanced Non-Small Cell Lung Cancer: A Multi-Institutional Phase II Trial' CLINICAL CANCER RESEARCH vol. 10, 2004, pages 7860 - 7864, XP003000957
- BROKER L.E. ET AL.: 'The role of new agents in the treatment of non-small cell lung cancer' EUROPEAN JOURNAL OF CANCER vol. 38, 2002, pages 2347 - 2361, XP004395672

**Description**

Technical Field

The present invention relates to a radiotherapy enhancer that can reduce the radiation dose and adverse drug reactions, when used in combination with a specific cancer radiotherapy.

Background Art

Kelly S.A. et al, CLINICAL RADIOLOGY (1989) vol. 40(3), 311-313 discloses the use of simultaneous radiotherapy and 5-fluorouracil in patients with inoperable squamous cell lung cancer.

Conventionally, surgical therapy, chemotherapy, immunotherapy, thermotherapy, and radiotherapy have been performed for the treatment of cancer (malignant tumor). Radiotherapy is often performed for various types of cancers such as gastric cancer, colorectal cancer, pancreatic cancer, head and neck cancer, esophageal cancer, lung cancer, and breast cancer that are advanced to stage III or IV. However, long-term treatment using radiation alone (a total radiation dose of 40 to 60 Gy is currently used in clinical setting) is thought to be difficult due to adverse drug reactions in the digestive system, such as hematological toxicity and dry mouth, and its clinical effect (antitumor effect) is therefore insufficient. To achieve a high antitumor effect, chemoradiotherapy using chemotherapeutic drugs and radiation in combination has recently been introduced as one of standard therapies, and it is said that its treatment results are better than those of therapies using radiation alone or chemotherapy alone (Non-Patent Document 1). For example, it has been disclosed that a combination of carboplatin/fluorouracil and radiation (Non-Patent Document 2) or cisplatin and radiation (Non-Patent Document 3) for the treatment of head and neck cancer, a combination of fluorouracil/cisplatin and radiation (Non-Patent Document 4) for the treatment of esophageal cancer, a combination of fluorouracil and radiation (Non-Patent Document 5) for the treatment of pancreatic cancer, and a combination of cisplatin/vinblastine and radiation (Non-Patent Document 6) for the treatment of non-small cell lung cancer significantly prolong the survival time as compared with therapies using radiation alone. Furthermore, a report has shown that the recurrence rate was lower, and the survival time is longer in patients with rectal cancer who postoperatively underwent chemoradiotherapy than in patients who did not (Non-Patent Document 7). However, since adverse drug reactions of chemotherapeutic drugs themselves occur in the conventional use of chemotherapeutic drugs and radiotherapy in combination, the medial practice may have to be discontinued as a result. Satisfactory effect of reducing adverse drug reactions has not been obtained either.

Various attempts have been made to develop a radiation sensitizer that reduces the radiation dose and adverse drug reactions without compromising the therapeutic effect of radiotherapy. For example, certain types of nitroimidazole derivatives are known as radiation sensitizers, and compounds such as misonidazole and etanidazole have been developed. However, these compounds have not been used in practice due to their too severe neurotoxicity at doses at which sensitization activity can be obtained and the like. While combination use of a drug that enhances radiation sensitivity is desired in the treatment of radiation-resistant tumors, this neurotoxicity has become problematic in the development of many of the previously reported radiation sensitivity enhancers (radiation sensitizers, etc.).

[Non-Patent Document 1] International Journal of Clinical Oncology, Vol.9, No.6, (2004): 414-490
[Non-Patent Document 2] Calais et al., J. Natl. Cancer Inst. 91 (1999): 2081-2086
[Non-Patent Document 3] Jeremic B, et al., J. Clin. Oncol. 18 (2000): 1458-1464
[Non-Patent Document 4] Al-Sarraf M, et al., J. Clin. Oncol. 15 (1997): 277-284
[Non-Patent Document 5] Moertel CG, et al., Cancer 48 (1981): 1705-1710
[Non-Patent Document 6] Sause W, et al., Chest 117(2000): 358-364
[Non-Patent Document 7] Tveit KM, et al., Br. J. Cancer 84(1997): 1130-1135
[Non-Patent Document 8] Harada K, et al., Oral Oncology 40 (204): 713-719 discloses the combined effects of the oral fluoropyrimidine anticancer agent, S-1 and radiation on human oral cancer cells.
[Non-Patent Document 9] Iwase H, et al., Int. J. Clin. Oncol. 9 (2004): 398 - 402 discloses the treatment of esophageal cancer by chemoradiotherapy followed by chemotherapy with S-1 and cisplatin.
[Non-Patent Document 10] Takahashi T, et al., Jpn. J. Clin. Oncol. 33 (2003): 584 - 588 discloses a case of advanced gastric cancer treated by chemotherapy with S-1 plus low-dose cisplatin and radiation.
[Non-Patent Document 11] Kelly SA, Macleod PM, Ash DV, clinical Radiology 40 (1989): 311 - 313 discloses the use of simultaneous radiotherapy and 5-fluorouracil in patients with inoperable squamous cell lung cancer, wherein the degree of palliation and survival obtained was found to be no different to that of a control group treated with radiotherapy alone.

Disclosure of the Invention

However, satisfactory effect of reducing adverse drug reactions has not been obtained in conventional combination use of chemotherapeutic drugs and radiotherapy due to adverse drug reactions of chemotherapeutic drugs themselves.

Accordingly, an object of the present invention is to provide a radiotherapy enhancer that can reduce the radiation dose and adverse drug reactions when used in combination with cancer radiotherapy.

Accordingly, the inventors of the present invention investigated radiotherapy enhancing effects of various substances. As a result, they found that a composition comprising the following components (A) and (B), which are known as antitumor agents, had an excellent radiotherapy enhancing effect and can reduce the radiation dose and adverse drug reactions when used in combination with radiotherapy, and accomplished the present invention.

That is, the present invention provides a radiotherapy enhancer comprising (A) tegafur and (B) gimeracil as further defined by claim 1.

Furthermore, the present invention discloses cancer radiotherapy **characterized in that** the above-mentioned radiotherapy enhancer and radiation are used in combination. The cancer radiotherapy of the present invention is for the treatment of lung cancer and pancreatic cancer.

Furthermore, the present invention provides use of (A) tegafur and (B) gimeracil for the production of a radiotherapy enhancer as further defined by claim 2.

Since combination use of the radiotherapy enhancer of the present invention and radiotherapy achieves excellent cancer therapeutic effect at a lower radiation dose and reduces adverse drug reactions, long-term effective cancer treatment is enabled.

Brief Description of the Drawing

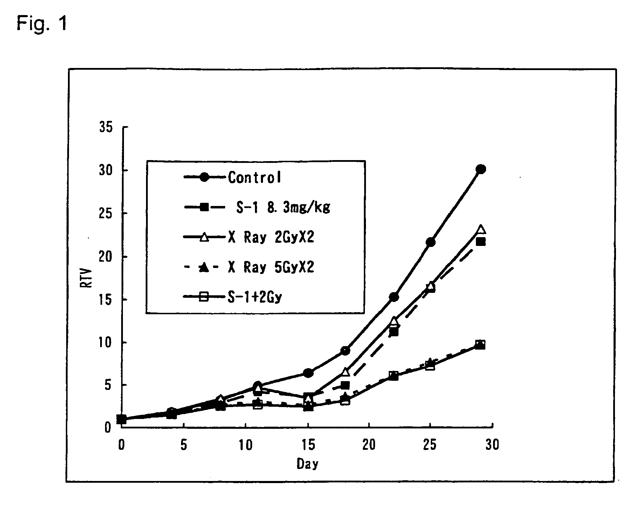

Figure 1 shows the tumor volume ratios (relative tumor volumes) to the initial tumor volumes.

Best Mode for Carrying Out the Invention

Tegafur, component (A) used in the radiotherapy enhancer of the present invention, is a 5-fluorouracil (hereinafter, referred to as "5-FU") prodrug, which is activated in an organism to release 5-FU, the main body of activity, and is known as an excellent antitumor agent with reduced toxicity, adverse drug reactions as compared to 5-FU. Gimeracil, component (B) used in the radiotherapy enhancer of the present invention can be produced by, for example, the method described in Japanese Unexamined Patent Publication No. 62-155215. Gimeracil is known to have an action of retaining 5-FU in blood and tumor tissues at high concentrations for a long period by selectively inhibiting dihydropyrimidine dehydrogenase (DPD), a 5-FU-catabolizing enzyme abundantly distributed in the liver. However, it is not known that these compositions have a radiotherapy enhancing effect, in the context of the present invention.

The mixture ratio of component (A) and (B) by mole is 1:0.4.

Combination use of a composition comprising the components (A) and (B) and radiotherapy markedly enhances the cancer therapeutic effect of radiation compared with radiotherapy alone. Therefore, this composition is useful as a radiotherapy enhancer. Furthermore, since an adequate therapeutic effect on cancer can be obtained at a lower radiation dose as a result of the enhanced effect of radiotherapy, this composition can also act as an agent for reducing the radiation dose in cancer treatment. Furthermore, since prolonged high-dose radiotherapy causes adverse drug reactions such as hematological toxicity, digestive toxicity, anorexia, malaise, and body weight loss, some patients could not receive long-term treatment previously. However, since combination of this composition and radiotherapy can reduce the radiation dose and hence reduces these adverse drug reactions, longer-term radiotherapy is enabled, resulting in improved therapeutic effects on cancer. Furthermore, radiotherapy causes severe dermatitis in the skin at the radiation-irradiated site, with skin disorders such as redness, dryness, skin abrasion, blister, and erosion, and may cause pigmentation, joint contracture, swelling of extremities, and the like later. However, combination use of this composition can prevent or relieve skin adverse drug reactions of radiation. Therefore, this composition is also useful as an agent for preventing or relieving adverse drug reactions of radiation, particularly as an agent for preventing or relieving skin adverse drug reactions of radiation.

The term "radiotherapy enhancer" used in the present specification refers to a drug that enhances (improves) radiation sensitivity (also referred to as radiation sensitivity enhancer, radiation sensitizer, or radiation sensitizing agent) irrespective of the mechanism of action.

Furthermore, radiotherapy intended in the present invention is commonly used in this technical field and can be performed according to protocols known to those skilled in the art. For example, irradiation with cesium, iridium, iodine, or cobalt is included in the above-mentioned radiotherapy. Radiotherapy may be systemic irradiation (for the treatment of some solid cancers), but local irradiation of tumor sites or tissues (irradiation of the abdomen, lungs for solid cancers) is preferred. Radiotherapy is commonly divided into 25 to 30 fractions (over about 5 to 6 weeks) and performed for 2 to 3 minutes per day.

The radiotherapy enhancer of the present invention can be used as an auxiliary agent in a radiotherapy of malignant tumors that are not originally sensitive to radiation or have acquired radiation resistance as a result of radiotherapy. Furthermore, the radiotherapy enhancer of the present invention can reduce the radiation dose used in the therapy by enhancing the radiation sensitivity of tumor cells (can reduce the dose to 1/2 to 1/3 of the conventional dose, for example). Therefore, adverse drug reactions due to radiation injury inevitably associated with radiotherapy (for example, stomatitis, myelopathy, radiation ulcer, radiation pneumonia, skin disorders, etc.) can be reduced. Furthermore, since the treatment period (exposure time) can be made longer than a period specified in usual protocols (can be prolonged 1.5- to 2-fold, for example), an excellent antitumor effect can be obtained.

The radiotherapy enhancer of the present invention is administered at the time of radiotherapy, either before or after radiotherapy. Furthermore, since the radiation enhancer of the present invention enhances the effect of radiotherapy as described above, it may be used in combination with other antitumor agents. Examples of such antitumor agents include platinum drugs, taxane drugs, vinca alkaloid drugs, topoisomerase inhibitors, antimetabolites, alkylating agents. More specific examples include one type or two or more types of antitumor agents such as cisplatin, carboplatin, oxaliplatin, Taxol, Taxotere, vincristine, vinblastine, vinorelbine, vindesine, irinotecan hydrochloride, topotecan, etoposide, teniposide, doxorubicin, gemcitabine, cytarabine, methotrexate, Alimta, cyclophosphamide, adriamycin, and mitomycin. These antitumor agents are used in combination, taking into account the patient's age and sex, severity of symptoms/adverse drug reactions, drug incompatibility.

Furthermore, the radiotherapy enhancer of the present invention can further contain oxonic acid or a pharmacologically acceptable salt thereof to reduce adverse drug reactions such as inflammation in the gastrointestinal tract or diarrhea caused by oral administration of the composition. Keto-enol isomers of oxonic acid, i.e., 1,4,5,6-tetrahydro-4,6-dioxo-1,3,5-triazine-2-carboxylic acid naturally fall within the scope of the present invention. Salts of oxonic acid include both pharmacologically acceptable acid addition salts and basic compound salts. Examples of acids that can form acid addition salts include inorganic acids such as hydrochloric acid, sulfuric acid, phosphoric acid, and hydrobromic acid and organic acids such as oxalic acid, succinic acid, maleic acid, fumaric acid, malic acid, tartaric acid, citric acid, malonic acid, methanesulfonic acid, and benzoic acid. Furthermore, examples of basic compounds that can form pharmacologically acceptable basic compound salts include sodium hydroxide, potassium hydroxide, calcium hydroxide, sodium carbonate, potassium hydrogencarbonate. Of these, potassium salts are particularly preferred. Furthermore, substances that produce oxonic acid in an organism may be used as oxonic acid. The mixture ratio of oxonic acid or a pharmacologically acceptable salt thereof in the present invention composition is about 0.1 to 5 moles, preferably about 0.2 to 2 moles, more preferably about 1 mole based on the component (A). A mole ratio of component (A):component (B):oxonic acid or a pharmacologically acceptable salt thereof = 1:0.4:1 is particularly preferred. Furthermore, the amount added to the drug of the present invention is suitably selected depending on the dosing regimen, the patient's age, sex, and other conditions, severity of the disease, and it is usually preferable for oral administration that the daily dose per kg of body weight is about 0.1 to 100 mg, preferably about 0.5 to 40 mg.

The radiotherapy enhancer of the present invention can be produced in the form of a usual pharmaceutical preparation using pharmaceutically acceptable carriers such as, for example, fillers, extenders, binders, moisturizing agents, disintegrating agents, surfactants, lubricants, and excipients. Examples of this pharmaceutical preparation include tablet, pill, powder, solution, suspension, emulsion, granule, capsule, suppository, injection (solution, suspension), ointment. The radiotherapy enhancer of the present invention can be prepared in the form of tablet using, for example, excipients such as lactose, sucrose, sodium chloride, glucose, urea, starch, calcium carbonate, kaolin, crystalline cellulose, and silicic acid, binders such as water, ethanol, propanol, simple syrup, glucose solution, starch solution, gelatin solution, carboxymethylcellulose, shellac, methylcellulose, potassium phosphate, and polyvinylpyrrolidone, disintegrating agents such as dry starch, sodium alginate, powdered agar, powdered laminaran, sodium hydrogencarbonate, calcium carbonate, polyoxyethylene sorbitan fatty acid esters, lauryl sodium sulfate, monoglyceride stearate, starch, and lactose, disintegration inhibitors such as sucrose, stearin, cocoa butter, and hydrogenated oils, absorption promoters such as quaternary ammonium base and lauryl sodium sulfate, moisturizing agents such as glycerine and starch, adsorbents such as starch, lactose, kaolin, bentonite, and colloidal silicic acid, lubricants such as purified talc, stearates, powdered boric acid, and polyethylene glycol. Furthermore, tablet can be coated with a usual coating as required to prepare, for example, a sugar-coated tablet, gelatin-encapsulated tablet, enteric-coated tablet, film coated tablet, double-layer tablet, or multilayer tablet. The radiotherapy enhancer of the present invention can be prepared in the form of pill using, for example, excipients such as glucose, lactose, starch, cacao butter, hydrogenated vegetable oil, kaolin, and talc, binders such as gum arabic powder, tragacanth powder, gelatin, and ethanol, disintegrating agents such as powdered laminaran and powdered agar. The radiotherapy enhancer of the present invention can be prepared in the form of suppository using, for example, polyethylene glycol, cacao butter, higher alcohols, higher alcohol esters, gelatin, semi-isynthesized glyceride. Capsule is prepared according to usual methods by usually mixing an active ingredient compound with various carriers mentioned above as examples and filling them in a hard gelatin capsule, soft capsule. When the radiotherapy enhancer of the present invention is prepared as an injection, the solution, emulsion, or suspension thereof is sterilized and is preferably isotonic with blood. When these forms are prepared, a wide variety of known diluents can be used, and examples thereof include water, ethyl alcohol, macrogol, propylene glycol, polyethoxylated isostearyl alcohol, polyoxyethylene sorbitan fatty acid esters. In this case, sodium chloride, glucose, or glycerine in an amount sufficient to prepare an isotonic solution may be contained in the pharmaceutical preparation, or usual solubilizing agents, buffers, soothing agents may be added. Furthermore, if necessary, coloring materials, preservatives, flavors, flavoring agents, sweeteners, or other drugs may be contained in the pharmaceutical preparation. The radiotherapy enhancer of the present invention can be prepared in the form of paste, cream, or gel by using white petrolatum, paraffin, glycerine, cellulose derivatives, polyethylene glycol, silicon, bentonite as a diluent.

The total amount of the above-described components (A) and (B) to be contained in the pharmaceutical preparation is not particularly limited and suitably selected in a wide range, but 1 to 70% by mass of the pharmaceutical preparation is usually desirable.

The administration method of the above-described pharmaceutical preparation is not particularly limited and determined depending on the dosage form, the patient's age, sex, and other conditions, severity of the disease. For example, oral administration as a tablet, pill, solution, suspension, emulsions, granule, or capsule is particularly preferred. The dose of the above-described pharmaceutical preparation is suitably selected depending on the dosing regimen, patient's age, sex, and other conditions, severity of the disease. The desired oral daily dose of component (A) as the active ingredient is usually about 0.1 to 100 mg per kg body weight, preferably about 0.5 to 30 mg, and the desired daily dose of component (B) is about 0.05 to 100 mg per kg body weight, preferably about 0.1 to 50 mg. Furthermore, the dose of the above-described pharmaceutical preparation can be divided and administered 1 to 4 times daily.

An excellent cancer treatment method can be provided by using the composition of the present invention and radiation in combination. Tumors for which this treatment method is used are pancreatic cancer and lung cancer.

Examples

The present invention will be explained more specifically with reference to the following test examples and comparative examples.

Test Example 1

(a) Preparation of test solution: Tegafur, gimeracil, and potassium oxonate were suspended in a 0.5% hydroxypropylmethylcellulose (HPMC) solution at concentrations of 0.83, 0.25, and 0.82 mg/mL, respectively, and the suspension was stirred at room temperature for about 10 minutes and ultrasonicated under iced water to obtain an S-1 drug solution of 8.3 mg/kg/day as tegafur. The dose of this S-1 drug solution is the no-observed adverse effect level when mice are orally given the solution for 14 consecutive days.

(b) Radiation (X-ray) irradiation method: Local irradiation was performed on human tumor strains transplanted into the right femoral region of the mouse using MBR-1505R Type 2 X-ray Irradiation System of Hitachi Medical Corporation under an irradiation condition (irradiation position) so that exposure per mouse should be 2 Gy or 5 Gy. To prevent systemic irradiation, mice were placed in a storage box made of lead so that only their right leg should be exposed to radiation.

(c) Test: The human lung cancer strains (LC-11 and Lu-99) subcutaneously transplanted into the back of a BALB/cA-nu mouse and grown beforehand were removed, cut into small fragments of about $2 \times 2$ mm$^2$ with scissors in physiological saline, and subcutaneously transplanted into the right femoral region of 5 to 6-week-old mice of the same strain with a transplantation needle. The mice were bred for at least 1 to 2 weeks and divided into the control group, the radiation alone group, the drug alone group, and the drug plus radiation group, so that the tumor volume and standard deviation (S.D.) in each group (n = 6 per group) should be as uniform as possible. Then, drug administration and X-ray irradiation were initiated. The drug treatment group was orally administered with 0.1 mL each of the above-described S-1 drug solution per body weight 10 g once daily for 14 consecutive days using a sonde for oral administration. The radiation group was irradiated with 2 Gy or 5 Gy of X-ray within about 1 hour after administration of the S-1 drug solution in the above-described manner on day 1, at the start of the test, and on day 8. Tumor-bearing mice in the control group (non-radiation/non-drug treatment group) and the radiation alone group were orally administered with 0.5% HPMC solution alone in the same manner for 14 consecutive days.

By using the following numerical formula 1, the tumor volume of each mouse in each group was obtained prior to the start of treatment experiment, on days 3, 5, 8 (1 week later) and 11 during the treatment period, and days 15 (2 weeks later),, 18, 22 (3 weeks later), 25, and 29 (4 weeks later) after completion of treatment. For the LC-11 strain, a relative tumor volume (RTV) to the tumor volume at the start of the test was obtained for each mouse. Figure 1 shows the mean RTV in each group as a tumor growth curve. For the Lu-99 strain, the mean tumor growth inhibition rate (%) in each treatment group based on the control group was obtained by using the following numerical formula 2 on days 15, at the end of the treatment period, and 29 at 4 weeks later and shown in Table 1.

(Numerical formula 1)

Tumor volume ($mm^3$) = (major axis) × (minor axis)$^2$ × 1/2

(Numerical formula 2)

Tumor growth inhibition rate (IR, %) = (1 - (mean tumor volume of treatment group)/(mean tumor volume of control group))

[Table 1]

Effect of combination use of S-1 drug solution and radiation (X-ray) on human lung cancer strain Lu-99

| Group number | Amount of S-1 drug solution (mg/kg) | Dose of X-ray irradiation (Gy) | IR(%) | |
|---|---|---|---|---|
| | | | Day 15 | Day 29 |
| 1 | 0 | 0 | - | - |
| 2 | 8.3 | 0 | 43.3 | 45.3 |
| 3 | 0 | 2 | 44.7 | 44.6 |
| 4 | 8.3 | 2 | 60.5* $ | 61.4* $ |
| 5 | 0 | 5 | 58.0 | 70.1 |

\* IUT test showed significant synergistic effect compared with treatment with S-1 drug solution alone (p = 0.0119)
$ Effect was significantly enhanced compared with 2-Gy radiation group (p < 0.01)

(d) Test results: X-ray irradiations on the LC-11 tumor strain at doses of 2 Gy and 5 Gy showed antitumor effects of 45.5% and 58.3%, respectively, on day 15 and 23% and 58%, respectively, on day 29. Treatment with the S-1 drug solution alone showed antitumor effects of 43% on day 15 and 28% on day 29. When used in combination with X-ray irradiation of 2-Gy, however, the S-1 drug solution significantly increased the antitumor effect of X-ray, with antitumor effects of 61% on day 15 and 68% on day 29. This effect is comparable to the antitumor effect of X-ray irradiation of 5-Gy alone. That is, it was found that low-dose X-ray irradiation exhibited an effect comparable to that of high-dose X-ray irradiation by using the composition of the present invention in combination. The examination using the LU-99 tumor strain also showed that X-ray irradiation of 2-Gy had exhibited antitumor effects of 44.7% on day 15 and 44.6% on day 29, and treatment with the S-1 drug solution alone had exhibited antitumor effects of 43.3% on day 15 and 45.3% on day 29, whereas use of X-ray irradiation of 2-Gy and the S-1 drug solution in combination had significantly enhanced the antitumor effects, with antitumor effects of 60.5% on day 15 and 61.4% on day 29. Since this antitumor effect of the combination use was comparable to the antitumor effect of 5-Gy irradiation alone (58.0% on day 15 and 70.1% on day 29), it was found that low-dose X-ray irradiation also exhibited an effect of high-dose X-ray irradiation by using the S-1 drug solution against this cancer strain. Furthermore, no serious adverse drug reactions such as body weight loss and skin disorders were observed in the mice receiving the S-1 drug solution and X-ray in combination.

Test Example 2

(a) Preparation of test solution I: Tegafur, gimeracil, and potassium oxonate were suspended in a 0.5% HPMC solution at concentrations of 0.83, 0.25, and 0.82 mg/mL, respectively, and the suspension was stirred at room temperature for about 10 minutes and ultrasonicated under iced water to obtain an S-1 drug solution of 8.3 mg/kg/day as tegafur. The dose of this S-1 drug solution is the no-observed adverse effect level when mice are orally given the solution for 14 consecutive days.

(b) Preparation of test solution II: Tegafur and uracil were suspended in a 0.5% HPMC solution at concentrations of 1.75 and 3.92 mg/mL, respectively, and the suspension was stirred with a stirrer at room temperature for 20 minutes and ultrasonicated under iced water to obtain a UFT drug solution of 17.5 mg/kg/day as tegafur. The dose of this UFT drug solution is the no-observed adverse effect level when mice are orally given the solution for 14 consecutive days.

(c) Radiation (X-ray) irradiation method: Local irradiation was performed on a human tumor strain transplanted into the right femoral region of the mouse using MBR-1505R Type 2 X-ray Irradiation System of Hitachi Medical Corporation under an irradiation condition (irradiation position) so that exposure per mouse should be 2 Gy. To prevent systemic irradiation, mice were placed in a storage box made of lead so that only their right leg should be exposed to radiation.

(d) Test: The human lung cancer strain LC-11 subcutaneously transplanted into the back of a BALB/cA-nu mouse and grown beforehand were removed, cut into small fragments of about $2 \times 2$ mm$^2$ with scissors in physiological saline, and subcutaneously transplanted into the right femoral region of 5 to 6-week-old mice of the same strain with a transplantation needle. The mice were bred for at least 1 to 2 weeks and divided into the control group, the radiation alone group, the drug alone group, and the drug plus radiation group, so that the tumor volume and standard deviation (S.D.) in each group (n = 6 per group) should be as uniform as possible. Then, drug administration and X-ray irradiation were initiated. The drug treatment group was orally administered with 0.1 mL each of the above-described S-1 and UFT drug solutions per body weight 10 g once daily for 14 consecutive days using a sonde for oral administration. The radiation group was irradiated with 2 Gy of X-ray within about 1 hour after administration of the S-1 or UFT drug solution in the above-described manner on day 1, at the start of the test, and on day 8. Tumor-bearing mice in the control group (non-radiation/non-drug treatment group) and the radiation alone group were orally administered with 0.5% HPMC solution alone in the same manner for 14 consecutive days.

By using the above-mentioned numerical formula 1, the tumor volume of each mouse in each group was obtained prior to the start of treatment experiment, on days 3, 5, 8 (1 week later) and 11 during the treatment period, and days 15 (2 weeks later), 18, 22 (3 weeks later), 25, and 29 (4 weeks later) after completion of treatment. A relative tumor volume (RTV) to the tumor volume at the start of the test was obtained for each mouse in each group. Then, the mean tumor growth inhibition rate (IR;%) in each treatment group based on the control group was obtained by using the above-mentioned numerical formula 2 on days 15, at the end of the treatment period, 22, and 29, at 4 weeks later, and shown in Table 2.

[Table 2]

Effect of combination use of S-1 and UFT drug solutions and X-ray irradiation on human lung cancer strain LC-11

| Group number | Dose of X-ray irradiation (Gy) | S-1 (mg/kg) | UFT (mg/kg) | IR (%) | | |
|---|---|---|---|---|---|---|
| | | | | Day 15 | Day 22 | Day 29 |
| 1 | - | - | - | - | - | - |
| 2 | 2 | - | - | 23.1 | 37.5 | 37.2 |
| 3 | - | 8.3 | - | 37.6 | 41.7 | 56.1 |
| 4 | 2 | 8.3 | - | 58.9 | 59.3 | 70.1 |
| 5 | - | - | 17.5 | 46.9 | 40.3 | 48.9 |
| 6 | 2 | - | 17.5 | 46.2 | 31.8 | 47.1 |
| 7 | 5 | - | - | 33.0 | 55.2 | 64.6 |

(e) Test results: Effects of uses of the S-1 drug solution (8.3 mg/kg) and UFT drug solution (17.5 mg/kg) at the no-observed adverse effect level in mice in combination with X-ray irradiation were compared. As a result, the use of the S-1 drug solution in combination with X-ray (2 Gy) significantly enhanced antitumor effects compared with the use of the drug alone, whereas the use of the UFT drug solution in combination with X-ray irradiation hardly enhanced antitumor effects. That is, it appeared that the composition of the present invention had a radiotherapy enhancing effect, and the action of UFT drug solution was very weak. Furthermore, no serious adverse drug reactions such as body weight loss and skin disorders were observed in the mice receiving the S-1 drug solution and X-ray in combination.

Comparative Example 1 (radiotherapy enhancing effect of cisplatin)

Combination therapy using radiation and cisplatin is one of therapies commonly used in the clinical setting for the treatment of lung cancer. The effect of cisplatin in the combination therapy was verified.
(a) Preparation of test solution I: The cisplatin solution (0.5 mg/mL) available from Bristol-Myers Squibb Company was used as it was. 0.1 mL per mouse body weight 10 g was administered for the dose of cisplatin 5 mg/kg, and 0.125 mL per mouse body weight 10 g was administered for the dose of 7.5 mg/kg.

(b) Radiation (X-ray) irradiation method: Local irradiation was performed on a human tumor strain transplanted into the right femoral region of the mouse using MBR-1505R Type 2 X-ray Irradiation System of Hitachi Medical Corporation under an irradiation condition (irradiation position) so that exposure per mouse should be 2 Gy or 5 Gy. To prevent systemic irradiation, mice were placed in a storage box made of lead so that only their right leg should be exposed to radiation.

(c) Test: The human lung cancer LC-11 strain subcutaneously transplanted into the back of a BALB/cA-nu mouse and grown beforehand were removed, cut into small fragments of about $2 \times 2$ mm$^2$ with scissors in physiological saline, and subcutaneously transplanted into the right femoral region of 5 to 6-week-old mice of the same strain with a transplantation needle. The mice were bred for at least 1 to 2 weeks and divided into the control group, the radiation alone group, the drug alone group, and the drug plus radiation group, so that the tumor volume and standard deviation (S.D.) in each group (n = 6 per group) should be as uniform as possible. Then, drug administration and X-ray irradiation were initiated. For the drug treatment group, 0.1 mL per body weight 10 g of a cisplatin solution for the dose of 5 mg/kg or 0.125 mL per body weight 10 g of this solution for the dose of 7.5 mg/kg was administered into the caudal vein on day 1. The radiation group was irradiated with 2 Gy of X-ray in the above-described manner on day 1, at the start of the test, and on day 8. For tumor-bearing mice in the control group (non-radiation/non-drug treatment group) and the radiation alone group, physiological saline was administered into the caudal vein on day 1.

By using the above-mentioned numerical formula 1, the tumor volume of each mouse in each group was obtained prior to the start of treatment experiment, on days 3, 5, 8 (1 week later) and 11 during the treatment period, and days 15 (2 weeks later), 18, 22 (3 weeks later), 25, and 29 (4 weeks later) after completion of treatment. A relative tumor volume (RTV) to the tumor volume at the start of the test was obtained for each mouse. Then, the mean tumor growth inhibition rate (IR; %) in each treatment group based on the control group was obtained by using the above-mentioned numerical formula 2 on day 15, at the end of the treatment period, and on day 29, at 4 weeks later, and shown in Table 3.

[Table 3]

X-ray irradiation enhancing effect of cisplatin

| Group number | Dose of X-ray irradiation (Gy) | Amount of CDDP (mg/kg) | IR (%) | |
|---|---|---|---|---|
| | | | Day 15 | Day 29 |
| 1 | - | - | - | - |
| 2 | 2 | - | 37.5 | 32.3 |
| 3 | - | 5.0 | 39.9 | 45.6 |
| 4 | 2 | 5.0 | 53.8 | 46.8 |
| 5 | - | 7.5 | 55.8 | 60.2 |
| 6 | 2 | 7.5 | 54.6 | 66.8 |
| 7 | 5 | - | 45.1 | 59.9 |

(c) Test results: Combination use of cisplatin 5 mg/kg or 7.5 mg/kg and X-ray irradiation of 2-Gy did not notably enhance antitumor effects compared with treatment with cisplatin alone, and the radiotherapy enhancing effect of cisplatin appeared to be very weak in a series of examinations using the human lung cancer LC-11 strain.

Test Example 3

(a) Preparation of test solution I: Tegafur, gimeracil, and potassium oxonate were suspended in a 0.5% hydroxypropylmethylcellulose (HPMC) solution at concentrations of 0.70, 0.21, and 0.79 mg/mL, respectively, and the suspension was stirred at room temperature for about 10 minutes and ultrasonicated under iced water to obtain an S-1 drug solution of 7.0 mg/kg/day as tegafur. The dose of this S-1 drug solution is the no-observed adverse effect level when a PAN-1 tumor-transplanted mouse is orally administered with the solution for 14 consecutive days.

(b) Preparation of test solution II: 5-fluorouracil (5-FU: Wako Pure Chemical Industries, Ltd.) was dissolved in physiological saline at a concentration of 1.5 mg/mL and sterilized by filtration with a 0.45-micron Millipore filter to obtain a drug solution of 15 mg/kg as 5-FU. The dose of this 5-FU drug solution is the maximum nontoxic dose when a PAN-4 tumor-transplanted mouse is intravenously administered with the solution on days 1 and 8.

(c) Preparation of test solution 3: Gemcitabine (2'-difluoro-2',3'-dideoxycytidine: Sigma) was dissolved in physiological saline at a concentration of 5 mg/mL and sterilized by filtration with a 0.45-micron Millipore filter to obtain a drug solution of 50 mg/kg as gemcitabine. The dose of this gemcitabine drug solution is the no-observed adverse effect level when a PAN-4 tumor-transplanted mouse is administered intravenously with the solution on days 1 and 8.

(d) Radiation (X-ray) irradiation method: Local irradiation was performed on a human tumor strain transplanted into the right femoral region of the mouse using MBR-1505R Type 2 X-ray Irradiation System of Hitachi Medical Corporation under an irradiation condition (irradiation position) so that exposure per mouse should be 2 Gy or 5 Gy. To prevent systemic irradiation, mice were placed in a storage box made of lead so that only their right leg should be exposed to radiation.

(e) Test: The human pancreatic cancer strain (PAN-4) subcutaneously transplanted into the back of a BALB/cA-nu mouse and grown beforehand was removed, cut into small fragments of about $2 \times 2$ mm$^2$ with scissors in physiological saline, and subcutaneously transplanted into the right femoral region of 5 or 6-week-old mice of the same strain with a transplantation needle. The mice were prebred for at least 1 to 2 weeks and divided into the control group, the radiation alone group, the drug alone group, and the drug plus radiation group, so that the tumor volume and standard deviation (S.D.) in each group (n = 6 per group) should be as uniform as possible. Then, drug administration and X-ray irradiation were initiated. For the S-1 drug solution, the drug treatment group was orally administered with 0.1 mL of the above-described S-1 drug solution per body weight 10 g once daily for 14 consecutive days using a sonde for oral administration. For 5-FU and gemcitabine, the drug treatment group was administered intravenously with 0.1 mL of the above-described 5-FU and gemcitabine drug solutions per body weight 10 g using a syringe for intravenous infusion on days 1 and 8. The radiation group was irradiated with 2 Gy or 5 Gy of X-ray in the above-described manner within about 1 hour after administration of each drug solution on day 1, at the start of the test, and on day 8. Tumor-bearing mice in the control group (non-radiation/non-drug treatment group) and the radiation alone group were orally administered with 0.5% HPMC solution alone in the same manner for 14 consecutive days.

By using the above-mentioned numerical formula 1, the tumor volume of each mouse in each group was obtained prior to the start of treatment experiment, on days 3, 5, 8 (1 week later) and 11 during the treatment period, and days 15 (2 weeks later), 18, 22 (3 weeks later), 25, and 29 (4 weeks later) after completion of treatment. For the PAN-4 strain, a relative tumor volume (RTV) to the tumor volume at the start was obtained. The mean tumor growth inhibition rate (%) in each treatment group based on the control group was obtained by using the above-mentioned numerical formula 2 on days 15, at the end of the treatment period, 22, and 29 and shown in Table 4.

[Table 4]

Effect of combination use of X-ray on human pancreatic cancer strain PAN-4

| Group | Dose of X-ray irradiation (Gy) | Amount of drug solution (mg/kg) | N | Tumor growth inhibition rate (IR) (%) | | |
|---|---|---|---|---|---|---|
| | | | | Day 15 | Day 22 | Day 29 |
| X-ray | 2 | - | 6 | 12.3 | 19.7 | 26.5 |
| | 5 | - | 6 | 36.1 | 61.3 | 61.4 |
| S-1 | - | 7.0 | 6 | 17.0 | 38.2 | 40.4 |
| S-1+X-ray | 2 | 7.0 | 6 | 39.9 | 56.0 | 68.3 |
| 5-FU | - | 15 | 6 | 12.4 | 16.8 | 29.2 |
| 5-FU+X-ray | 2 | 15 | 6 | 15.8 | 27.5 | 36.9 |
| Gemcitabine | - | 50 | 6 | 39.8 | 51.1 | 51.1 |
| Gemcitabine+X-ray | 2 | 50 | 6 | 40.0 | 62.3 | 63.9 |

(f) Test results: X-ray irradiation at doses of 2 Gy and 5 Gy on the PAN-4 tumor strain showed antitumor effects of 12.3% and 36.1%, respectively, on day 15, 19.7% and 61.3%, respectively, on day 22, and 26.5% and 61.4%, respectively, on day 29. Treatment with the S-1 drug solution alone showed antitumor effects of 17.0% on day 15, 38.2% on day 22, and 40.4% on day 29. However, when used in combination with X-ray irradiation of 2-Gy, the S-1 drug solution significantly increased the antitumor effect of X-ray, with antitumor effects of 39.9% on day 15, 56% on day 22, and 68.3% on day 29. This effect is comparable to the antitumor effect of X-ray irradiation of 5-Gy alone, that is, it was found that low-dose X-ray irradiation achieved an effect of high-dose X-ray irradiation by using the composition of the present invention. Furthermore, no serious adverse drug reactions such as body weight loss and skin disorders were observed in mice of the S-1 drug solution plus X-ray group. On the other hand, treatment with 5-FU alone showed antitumor effects of 12.4% on day 15, 16.8% on day 22, and 29.2% on day 29, and, even when used in combination with X-ray irradiation of 2-Gy, did not show marked effect of the combination use, with effects of 15.8% on day 15, 27.5% on day 22, and 36.9% on day 29. Furthermore, treatment with gemcitabine alone showed antitumor effects of 39.8% on day 15, 51.1% on day 22, and 51.1% on day 29 and, even when used in combination with X-ray irradiation of 2-Gy, did not show a strong effect of the combination use, with effects of 40% on day 15, 62.3% on day 22, and 63.9% on day 29.

The above results suggested that the combination therapy using the S-1 drug solution and radiation against the human pancreatic cancer strain was more effective than the combination therapy using 5-FU and radiation or gemcitabine and radiation, which is being performed in clinical practice, and therefore was a useful therapy.

Preparation Example 1: Tablets

| | |
|---|---|
| Tegafur | 30 mg |
| Gimeracil | 18 mg |
| Starch | 110 mg |
| Magnesium stearate | 17 mg |
| Lactose | 40 mg |
| Total | 215 mg |

Tablets of 215 mg/tablet were prepared with the above mixture composition according to a usual method.

Preparation Example 2: Tablets

| | |
|---|---|
| Tegafur | 50 mg |
| Gimeracil | 8 mg |
| Lactose | 45 mg |
| Crystalline cellulose | 20 mg |
| Magnesium stearate | 5 mg |
| Talc | 4 mg |
| Methylcellulose | 10 mg |
| Total | 142 mg |

Tablets of 142 mg/tablet were prepared with the above mixture composition according to a usual method.

Preparation Example 3: Tablets

| | |
|---|---|
| Tegafur | 40 mg |
| Gimeracil | 12 mg |
| Potassium oxonate | 39 mg |
| Lactose | 54 mg |
| Crystalline cellulose | 20 mg |
| Magnesium stearate | 5 mg |

(continued)

| | |
|---|---|
| Talc | 3 mg |
| Methylcellulose | 10 mg |
| Total | 183 mg |

Tablets of 183 mg/tablet were prepared with the above mixture composition according to a usual method.

Preparation Example 4: Granules

| | |
|---|---|
| Tegafur | 200 mg |
| Gimeracil | 58 mg |
| Lactose | 340 mg |
| Corn starch | 450 mg |
| Hydroxypropylmethylcellulose | 10 mg |
| Total | 1058 mg |

Granules were prepared with the above mixture composition according to a usual method.

Preparation Example 5: Suppository

| | |
|---|---|
| Tegafur | 300 mg |
| Gimeracil | 110 mg |
| Witepsol W-35 | 900 mg |
| Total | 1310 mg |

A suppository was prepared with the above mixture composition according to a usual method.

## Claims

1. A radiotherapy enhancer comprising

    (A) tegafur and
    (B) gimeracil

    for use in combination with a radiotherapy for the treatment of lung cancer or pancreatic cancer,
    wherein a mixture ratio by mole of components (A) and (B) is 1:0.4.

2. Use of (A) tegafur and (B) gimeracil for a production of a radiotherapy enhancer for treatment of lung cancer or pancreatic cancer in combination with a radiotherapy,
    wherein a mixture ratio by mole of the components (A) and (B) in the radiotherapy enhancer is 1:0.4.

## Patentansprüche

1. Radiotherapieverstärker, umfassend

    (A) Tegafur und
    (B) Gimeracil

    zur Verwendung in Kombination mit einer Radiotherapie zur Behandlung von Lungenkrebs oder Bauchspeicheldrüsenkrebs, wobei das Mischungsverhältnis nach Molen der Komponenten (A) und (B) 1:0,4 beträgt.

2. Verwendung von (A) Tegafur und (B) Gimeracil zur Herstellung eines Radiotherapieverstärkers zur Behandlung von Lungenkrebs oder Bauchspeicheldrüsenkrebs in Kombination mit Radiotherapie, wobei ein Mischungsverhältnis nach Molen der Komponenten (A) und (B) in dem Radiotherapieversärker 1:0,4 beträgt.

**Revendications**

1. Potentialisateur pour radiothérapie comprenant

    (A) du tégafur et
    (B) du gimeracil

pour utilisation en combinaison avec une radiothérapie pour le traitement du cancer du poumon ou du cancer pancréatique,
dans lequel le rapport molaire du mélange des composants (A) et (B) est de 1 : 0,4.

2. Utilisation de tégafur (A) et de gimeracil (B) pour la production d'un potentialisateur pour radiothérapie pour le traitement du cancer du poumon ou du cancer pancréatique en combinaison avec une radiothérapie, le rapport molaire du mélange des composants (A) et (B) au sein du potentialisateur de radiothérapie étant de 1 : 0,4.

Fig. 1

# EP 1 864 683 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 62155215 A

### Non-patent literature cited in the description

- **Kelly S.A. et al.** *CLINICAL RADIOLOGY,* 1989, vol. 40 (3), 311-313
- *International Journal of Clinical Oncology,* 2004, vol. 9 (6), 414-490
- **Calais et al.** *J. Natl. Cancer Inst.,* 1999, vol. 91, 2081-2086
- **Jeremic B et al.** *J. Clin. Oncol.,* 2000, vol. 18, 1458-1464
- **Al-Sarraf M et al.** *J. Clin. Oncol.,* 1997, vol. 15, 277-284
- **Moertel CG et al.** *Cancer,* 1981, vol. 48, 1705-1710
- **Sause W et al.** *Chest,* 2000, vol. 117, 358-364
- **Tveit KM et al.** *Br. J. Cancer,* 1997, vol. 84, 1130-1135
- **Harada K et al.** *Oral Oncology,* vol. 40, 713-719
- **Iwase H et al.** *Int. J. Clin. Oncol.,* 2004, vol. 9, 398-402
- **Takahashi T et al.** *Jpn. J. Clin. Oncol.,* 2003, vol. 33, 584-588
- **Kelly SA ; Macleod PM ; Ash DV.** *clinical Radiology,* 1989, vol. 40, 311-313